# EUROPEAN PATENT SPECIFICATION

(11) **EP 3 824 057 B1**
(45) Date of publication and mention of the grant of the patent: **18.10.2023**
(21) Application number: 19736751.9
(22) Date of filing: 11.07.2019
(51) Int. Cl.: C11D 1/83, A61K 8/60, A61Q 19/10, C11D 1/37, C11D 1/66, C11D 1/04

(54) **USE OF A RHAMNOLIPID IN A SURFACTANT SYSTEM**
VERWENDUNG EINES RHAMNOLIPIDS IN EINEM TENSIDSYSTEM
UTILISATION D'UN RHAMNOLIPIDE DANS UN SYSTÈME TENSIOACTIF

(30) Priority: 17.07.2018 EP 18183977
(43) Date of publication of application: 26.05.2021
(73) Proprietor: Unilever Global IP Limited, Wirral, Merseyside CH62 4ZD (GB); Unilever IP Holdings B.V., 3013 AL Rotterdam (NL)
(72) Inventor: STEVENSON, Paul, Simon, Wirral Merseyside CH63 3JW (GB); PARRY, Neil, James, Wirral Merseyside CH63 3JW (GB); JAISWAL, Prem, Kumar, 26841 Casalpusterlengo Lodi (IT); VACCHELLI, Valeria, 26841 Casalpusterlengo Lodi (IT)
(74) Representative: McHugh, Paul Edward
(86) International application number: PCT/EP2019/068688
(87) International publication number: WO 2020/016097

(56) References cited:
- EP-A1- 2 786 742
- EP-A1- 2 786 743
- EP-A1- 3 290 500
- WO-A1-2014/095367
- WO-A1-2016/096478
- WO-A2-2014/118095
- US-A1- 2014 349 902
- US-A1- 2016 045 424
- US-A1- 2017 071 835
- US-A1- 2018 023 040
- US-A1- 2018 044 612
- US-A1- 2018 044 614
- Anonymous: "Bio-friendly Replacement to Conventional Surfactants Now Available for Sale | Business Wire", , 9 December 2015 (2015-12-09), XP055921808, Retrieved from the Internet: URL:https://www.businesswire.com/news/home /20151209005166/en/Bio-friendly-Replacemen t-to-Conventional-Surfactants-Now-Availabl e-for-Sale [retrieved on 2022-05-17]

## Description

### Field of Invention

The invention concerns the use of a rhamnolipid in a surfactant system for handwash detergents.

### Background of the Invention

Handwash detergents are detergents that involve the consumer using their hands to wash substrates. Fields of use principally involve laundry use (i.e. the hand washing of clothes) and hand dish wash (HDW) (i.e. the hand washing of dishes). Handwash detergents involve intimate contact of the detergent liquor with the hands during the washing process, whether in laundry or HDW. Although formulation efficacy is an important attribute for returning consumer use, the sensorial experience of the formulation and it's use in the washing process by the consumer both during, and immediately after formulation use leaves a lasting impression on whether the consumer has had a good or bad experience with the product.

Sensorial experiences can include for example the pleasant colour or fragrance of the product both by itself or during use. Another important sensorial experience for consumers using handwash detergents is the lasting sensorial impression left on the hands. This is important as the handwash process involves the consumer's hands being in contact with the formulation and resulting wash liquor, and the formulation can be harsh on the skin.

These is a need for improved handwash detergents that leave an improved sensorial impression left on the hands. In particular, those that leave an improved sensorial impression on the mildness on the hands, both during the washing process and a lasting improved sensorial impression on the mildness on the hands after the handwash process is ended.

One way of solving this is to include an ingredient to enhance mildness, such as a polyethylene glycol ingredient.

US 2018/044614 A1 and US 2018/044612 A1 are representative of the state of the art.

These documents disclose compositions comprising a rhamnolipid surfactant and an anionic surfactant different to said rhamnolipid surfactant.

EP 2786743 A1 discloses rhamnolipid compositions which have good foam properties and have a light skin feel when used in skin care compositions or skin cleansing compositions.

### Summary of the Invention

We have found that use of a rhamnolipid in a surfactant system for handwash detergents to confer to the consumer a sensorial impression of mildness on the hands. By using the rhamnolipid, the necessity for using ingredients such as polyethylene glycol is reduced or negated.

The invention relates to the use of a rhamnolipid in a surfactant system for handwash detergents to confer to the consumer a sensorial impression of mildness on the hands, wherein the rhamnolipid is present in the surfactant system at a level of from 15 to 50 wt.% of the surfactant system, wherein the use involves intimate contact of the detergent liquor with the hands during the washing process, whether in laundry processes or hand dish wash processes.

Preferably the sensorial impression of mildness on the hands lasts after the hands are subsequently dried after the handwash process is ended.

Preferably the handwash detergent is a fluid detergent composition, preferably an aqueous detergent composition.

Preferably the rhamnolipid is present in the composition at a level of from 1 to 20 wt.%, preferably from 1.25 to 15 wt.%, more preferably from 1.5 to 12.5 wt.%, most preferably from 2 to 10 wt.%.

Preferably the handwash detergent is a hand dish wash composition, or liquid laundry detergent composition for hand wash.

Preferably the rhamnolipid comprises at least 50 wt.% di-rhamnolipid, more preferably at least 60 wt.% di-rhamnolipid, even more preferably 70 wt.% di-rhamnolipid, most preferably at least 80 wt.% di-rhamnolipid.

Preferably the rhamnolipid is a di-rhamnolipid of formula: Rha2C₈₋₁₂C₈₋₁₂.

It is intended that any preferable subject matter described herein can be combined with any other subject matter, particularly combining 2 or more preferable subject matters.

### Detailed Description of the Invention

### Handwash

By handwash detergents is meant detergents that involve the consumer using their hands to wash substrates. Preferred handwash uses principally involve laundry use (i.e. the hand washing of clothes) and hand dish wash (HDW) (i.e. the hand washing of dishes).

Handwash detergents involve intimate contact of the detergent liquor with the hands during the washing process, whether in laundry processes or hand dish wash processes.

### Rhamnolipids

Rhamnolipids are a class of glycolipid. They are constructed of rhamnose combined with beta-hydroxy fatty acids. Rhamnose is a sugar. Fatty acids are ubiquitous in animals and plants.

Rhamnolipids are discussed in Applied Microbiology and Biotechnology (2010) 86:1323-1336 by E. Deziel *et al.* Rhamnolipids are produced by Glycosurf, AGAE Technologies and Urumqi Unite Bio-Technology Co., Ltd. Rhamnolipids may be produced by strains of the bacteria *Pseudomonas Aeruginosa.* Rhamnolipids may also be produced by a recombinant cell of *Pseudomonas Putida* where the recombinant cell comprises increased activity of at least one of the enzymes a/P hydrolase, rhamnosyltransferase I or rhamnosyl-transferase II compared to the wild-type of the cell.

There are two major groups of rhamnolipids; mono-rhamnolipids and di-rhamnolipids.

Mono-rhamnolipids have a single rhamnose sugar ring. A typical mono-rhamnolipid produced by P. aeruginosa is L-rhamnosyl-β-hydroxydecanoyl-β-hydroxydecanoate (RhaC,oC,o). It may be referred to as Rha-C₁₀-C₁₀, with a formula of C₂₆H₄₈O₉. Mono-rhamnolipids have a single rhamnose sugar ring.

The IUPAC Name is 3-[3-[(2R,3R,4R,5R,6S)-3,4,5-trihydroxy-6-methyloxan-2-yl]oxydecanoyloxy]decanoic acid.

Di-rhamnolipids have two rhamnose sugar rings. A typical di-rhamnolipid is L-rhamnosyl-L-rhamnosyl-β-hydroxydecanoyl-β-hydroxydecanoate (Rha2C₁₀C₁₀). It may be referred to as Rha-Rha-C-₁₀-C-₁₀, with a formula of C₃₂H₅₈O₁₃.

The IUPAC name is 3-[3-[4,5-dihydroxy-6-methyl-3-(3,4, 5-tri hyd roxy-6-m ethyloxan-2-yl)oxyoxan-2-yl]oxyd ecanoyloxy]decanoic acid.

In practice a variety of other minor components with different alkyl chain length combinations, depending upon carbon source and bacterial strain, exist in combination with the above more common rhamnolipids. The ratio of mono-rhamnolipid and di-rhamnolipid may be controlled by the production method. Some bacteria only produce mono-rhamnolipid, see US5767090: Example 1, some enzymes can convert mono-rhamnolipid to di-rhamnolipid.

In various publications mono-rhamnolipids have the notation Rha-, which may be abbreviated as Rh or RL2. Similarly, di-rhamnolipids have the notation Rha-Rha or Rh-Rh- or RL1. For historical reasons "rhamnolipid 2" is a mono-rhamnolipid and "rhamnolipid 1 " is a di-rhamnolipid. This leads to some ambiguity in the usage or "RL1 " and "RL2" in the literature.

Throughout this patent specification, we use the terms mono- and di-rhamnolipid in order to avoid this possible confusion. However, if abbreviations are used R1 is mono-rhamnolipid and R2 is di-rhamnolipid. For more information on the confusion of terminology in the prior art see the introduction to US 4814272.

The following rhamnolipids have been detected as produced by the following bacteria: (C12:1, C14:1 indicates fatty acyl chains with double bonds).

### Rhamnolipids produced by P. aeruginosa (mono-rhamnolipids):

Rha-C8-C10, Rha-C10-C8, Rha-C10-C10, Rha-C10-C12, Rha-C10-C12:1, Rha-C12-C10, Rha-C12:1-C10

### Rhamnolipids produced by P. aeruginosa (di-rhamnolipids):

Rha-Rha-C8-C10, Rha-Rha-C8-C12:1, Rha-Rha-C10-C8, Rha-Rha-C10-C10, Rha-Rha-C10-C12:1, Rha- Rha-C-10-C-12, Rha-Rha-C-12-C-10, Rha-Rha-C-12:1-C-12, Rha-Rha-C-10-C14:1

### Rhamnolipids produced by P. aeruginosa (unidentified as either mono- or di-rhamnolipids):

C8-C8, C8-C10, C10-C8, C8-C12:1, C12: 1-C8, C10-C10, C12-C10, C12:1-C10 C12-C12, C12: 1-C12, C14-C10, C14:1-C10, C14-C14.

### Rhamnolipids produced by P. chlororaphis (mono-rhamnolipids only):

Rha-C10-C 8, Rha-C10 -C10, Rha-C12-C10, Rha-C12:1-C10, Rha-C12-C12, Rha-C12: 1-C12, Rha-C14-C10. Rha-C-14:1-C-10.

Rhamnolipids produced by *Burkholdera pseudomallei* (di-rhamnolipids only): Rha-Rha-C14-C14.

Rhamnolipids produced by *Burkholdera* (Pseudomonas) plantarii (di-rhamnolipids only): Rha-Rha-C14-C14.

There are over 100 strains of P. aeruginosa on file at the American Type Culture Collection (ATCC). There are also a number of strains that are only available to manufacturers of commercial Rhamnolipids. Additionally there are probably thousands of strains isolated by various research institutions around the world. Some work has gone into typing them into groups. Each strain has different characteristics including how much rhamnolipid is produced, which types of rhamnolipids are produced, what it metabolizes, and conditions in which it grows. Only a small percentage of the strains have been extensively studied.

Through evaluation and selection, strains of *P. aeruginosa* can be isolated to produce rhamnolipids at higher concentrations and more efficiently. Strains can also be selected to produce less byproduct and to metabolize different feedstock or pollutants. This production is greatly affected by the environment in which the bacterium is grown.

Atypical di-rhamnolipid is L-rhamnosyl-L-rhamnosyl-β-hydroxydecanoyl-β-hydroxydecanoate (Rha₂C₁₀C₁₀ with a formula of C₃₂H₅₈O₁₃).

In practice a variety of other minor components with different alkyl chain length combinations, depending upon carbon source and bacterial strain, exist in combination with the above more common rhamnolipids. The ratio of mono-rhamnolipid and di-rhamnolipid may be controlled by the production method. Some bacteria only produce mono-rhamnolipid, see US5767090: Example 1, some enzymes can convert mono-rhamnolipid to di-rhamnolipid.

Preferably the rhamnolipid is present in the composition at a level of from 1 to 20 wt.%, preferably from 1.25 to 15 wt.%, more preferably from 1.5 to 12.5 wt.%, most preferably from 2 to 10 wt.%.

Preferably the rhamnolipid is selected from:
- Rhamnolipids produced by P. aeruginosa (mono-rhamnolipids):
   Rha-C8-C10, Rha-C10-C8, Rha-C10-C10, Rha-C10-C12, Rha-C10-C12:1, Rha-C12-C10, Rha-C12:1-C10
- Rhamnolipids produced by P. chlororaphis (mono-rhamnolipids only):
   Rha-C10-C8, Rha-C10-C10, Rha-C12-C10, Rha-C12:1-C10, Rha-C12-C12, Rha-C12:1-C12, Rha-C14-C10, Rha-C14:1-C10.
- Mono-rhamnolipids may also be produced from P.putida by introduction of genes rhlA and rhlB from Psuedomonas aeruginosa [Cha et al. in Bioresour Technol. 2008. 99(7):2192-9]
- Rhamnolipids produced by P. aeruginosa (di-rhamnolipids):
   Rha-Rha-C8-C10, Rha-Rha-C8-C12:1, Rha-Rha-C10-C8, Rha-Rha-C10-C10, Rha-Rha-C10-C12:1, Rha-Rha-C10-C12, Rha-Rha-C12-C10, Rha-Rha-C12:1-C12, Rha-Rha-C10-C14:1
- Rhamnolipids produced by Burkholdera pseudomallei (di-rhamnolipids only):
   Rha-Rha-C14-C14.
- Rhamnolipids produced by Burkholdera (Pseudomonas) plantarii (di-rhamnolipids only):
   Rha-Rha-C14-C14.
- Rhamnolipids produced by P. aeruginosa which are initially unidentified as either mono- or di-rhamnolipids:
   C8-C8, C8-C10, C10-C8, C8-C12:1, C12:1-C8, C10-C10, C12-C10, C12:1-C10, C12-C12, C12:1-C12, C14-C10, C14:1-C10, C14-C14.

Preferably the Rhamnolipid is L-rhamnosyl-β-hydroxydecanoyl-β-hydroxydecanoate (RhaC₁₀C₁₀ with a formula of C₂₆H₄₈O₉).

Preferably, the rhamnolipid comprises at least 50 wt.% di-rhamnolipid, more preferably at least 60 wt.% di-rhamnolipid, even more preferably 70 wt.% di-rhamnolipid, most preferably at least 80 wt.% di-rhamnolipid.

Preferably the rhamnolipid is a di-rhamnolipid of formula: Rha2C₈₋₁₂C₈₋₁₂. The preferred alkyl chain length is from C₈ to C₁₂. The alkyl chain may be saturated or unsaturated.

The most preferred di-rhamnolipid is an example of a di-rhamnolipid of formula: Rha2C₈₋₁₂C₈₋₁₂, known as Rhamnolipid R2 herein, and can be supplied from Evonik.

### Handwash detergent composition

The handwash detergent composition is a cleaning composition, useful for cleaning one or more substrates.

The composition is preferably a fluid detergent composition, more preferably an aqueous detergent composition.

Preferably the handwash detergent is a hand dish wash composition, or liquid laundry detergent composition for hand wash.

Preferably when a liquid laundry detergent, the hand dish wash composition when dissolved in demineralised water at 4g/L, 293K, has a pH of from 4 to 8, more preferably from 4.5 to 7.5.

Preferably when a liquid laundry detergent, the liquid laundry detergent composition when dissolved in demineralised water at 4g/L, 293K, has a pH of from 6 to 11, more preferably from 7 to 9.

### Second anionic surfactant

In the handwash composition, the composition comprises a second different anionic surfactant.

The composition comprises from 1 to 20 wt.%, preferably from 1.5 to 17.5 wt.%, more preferably from 2 to 15 wt.%, most preferably from 5 to 15 wt.% of a second different anionic surfactant.

In the handwash composition, preferably the rhamnolipid is present in the surfactant system at a level of from 10 to 90 wt.%, more preferably from 15 to 80 wt.%, most preferably from 15 to 50 wt.% of the surfactant system.

Preferably in the use aspect of the first aspect of the invention, the composition comprises a second different anionic surfactant.

When a second different anionic surfactant is used, preferably the rhamnolipid is present in the surfactant system at a level of from 10 to 90 wt.%, more preferably from 15 to 80 wt.%, most preferably from 15 to 50 wt.% of the surfactant system.

In all aspects of the invention, preferably the second anionic surfactant is selected from C₁₀ to C₂₀ linear alkylbenzene sulphonates, C₁₀ to C₂₀ alkyl sulphates, C₁₀ to C₂₀ alkyl ether sulphates, and mixtures thereof. More preferably the second different anionic surfactant is a mixture of anionic surfactants as previously specified. More preferably the second different anionic surfactant (b), is a mixture of C₁₀ to C₂₀ linear alkylbenzene sulphonates, and C₁₀ to C₂₀ alkyl ether sulphates. Most preferably the mixture of C₁₀ to C₂₀ linear alkylbenzene sulphonates, and C₁₀ to C₂₀ alkyl ether sulphates is a mixture at a ratio of from 10:90 to 90:10, preferably from 20:80 to 80:20, more preferably from 30:70 to 70:30.

### Additional Surfactants

Additional surfactants may be present in the composition.

Preferably the cleaning composition comprises from 0 to 20 wt.%, more preferably from 0 to 10 wt.% of additional surfactants.

These are preferably selected from further anionic, nonionic surfactants and zwitterionic surfactants.

In general, the nonionic and anionic surfactants of the surfactant system may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn, Carl Hauser Verlag, 1981. Preferably the surfactants used are saturated.

Preferred nonionic detergent compounds which may be used include the reaction products of compounds having a hydrophobic group and a reactive hydrogen atom, for example, aliphatic alcohols, acids, amides with alkylene oxides, especially ethylene oxide either alone or with propylene oxide. Specific nonionic detergent compounds are the condensation products of aliphatic primary or secondary linear or branched alcohols with ethylene oxide, generally 5 to 40 EO, preferably 7EO to 9EO.

Preferred anionic detergent compounds which may be used are usually water-soluble alkali metal salts of organic sulphates and sulphonates having alkyl radicals containing from 8 to 22 carbon atoms, the term alkyl being used to include the alkyl portion of higher acyl radicals. Examples of suitable synthetic anionic detergent compounds are sodium and potassium alkyl sulphates, especially those obtained by sulphating alcohols, produced for example from tallow or coconut oil, sodium and potassium alkyl C₁₀ to C₂₀ benzene sulphonates, particularly sodium linear secondary alkyl C₁₀ to C₁₅ benzene sulphonates; and sodium alkyl glyceryl ether sulphates, especially those ethers of the higher alcohols derived from tallow or coconut oil and synthetic alcohols derived from petroleum. The preferred anionic detergent compounds are sodium C₁₁ to C₁₅ alkyl benzene sulphonates and sodium C₁₂ to C₁₄ alkyl sulphates. Also applicable are surfactants such as those described in EP-A-328 177 (Unilever), which show resistance to salting-out, the alkyl polyglycoside surfactants described in EP-A-070 074, and alkyl monoglycosides.

Preferred surfactant systems are mixtures of anionic with nonionic detergent active materials, in particular, the groups and examples of anionic and nonionic surfactants pointed out in EP-A-346 995 (Unilever). Especially preferred is surfactant system that is a mixture of anionic surfactants as described earlier together with a C₁₂ to C₁₆ primary alcohol 3 to 7 EO ethoxylate.

Preferred zwitterionic surfactants include cocamidopropyl betaine. Preferred levels of zwitterionc surfactants are from 0.1 to 5 wt.%, preferably from 0.5 to 4 wt.%.

### Further Ingredients

The handwash compositions, depending on whether the handwash composition is a hand dish wash composition, or liquid laundry detergent composition for hand wash, may comprise any of these further preferred ingredients.

### Builders or Complexinq Agents

Builder materials may be particularly useful in liquid laundry detergent compositions for hand wash.

Builder materials may be selected from 1) calcium sequestrant materials, 2) precipitating materials, 3) calcium ion-exchange materials and 4) mixtures thereof.

Examples of calcium sequestrant builder materials include alkali metal polyphosphates, such as sodium tripolyphosphate and organic sequestrants, such as ethylene diamine tetra-acetic acid.

Examples of precipitating builder materials include sodium orthophosphate and sodium carbonate.

Examples of calcium ion-exchange builder materials include the various types of water-insoluble crystalline or amorphous aluminosilicates, of which zeolites are the best known representatives, e.g. zeolite A, zeolite B (also known as zeolite P), zeolite C, zeolite X, zeolite Y and also the zeolite P-type as described in EP-A-0,384,070.

The composition may also contain 0-65 % of a builder or complexing agent such as ethylenediaminetetraacetic acid, diethylenetriamine-pentaacetic acid, alkyl- or alkenylsuccinic acid, nitrilotriacetic acid or the other builders mentioned below. Many builders are also bleach-stabilising agents by virtue of their ability to complex metal ions.

Zeolite and carbonate (including bicarbonate and sesquicarbonate) are preferred builders.

The composition may contain as builder a crystalline aluminosilicate, preferably an alkali metal aluminosilicate, more preferably a sodium aluminosilicate. This is typically present at a level of less than 15 wt.%. Aluminosilicates are materials having the general formula:
0.8-1.5 M₂₀. Al₂O₃. 0.8-6 Si0₂
where M is a monovalent cation, preferably sodium. These materials contain some bound water and are required to have a calcium ion exchange capacity of at least 50 mg CaO/g. The preferred sodium aluminosilicates contain 1.5-3.5 SiO₂ units in the formula above. They can be prepared readily by reaction between sodium silicate and sodium aluminate, as amply described in the literature. The ratio of surfactants to alumuminosilicate (where present) is preferably greater than 5:2, more preferably greater than 3:1.

Alternatively, or additionally to the aluminosilicate builders, phosphate builders may be used. In this art the term 'phosphate' embraces diphosphate, triphosphate, and phosphonate species. Other forms of builder include silicates, such as soluble silicates, metasilicates, layered silicates (e.g. SKS-6 from Hoechst).

When a laundry composition, preferably the laundry detergent formulation is a non-phosphate built laundry detergent formulation, i.e., contains less than 1 wt.% of phosphate. Preferably the laundry detergent formulation is carbonate built if a builder is included.

### Fluorescent Agent

These materials may be particularly useful in liquid laundry detergent compositions for hand wash.

The composition preferably comprises a fluorescent agent (optical brightener).

Fluorescent agents are well known and many such fluorescent agents are available commercially. Usually, these fluorescent agents are supplied and used in the form of their alkali metal salts, for example, the sodium salts. The total amount of the fluorescent agent or agents used in the composition is generally from 0.005 to 2 wt.%, more preferably 0.01 to 0.1 wt.%. Preferred classes of fluorescer are: Di-styryl biphenyl compounds, e.g. Tinopal (Trade Mark) CBS-X, Di-amine stilbene di-sulphonic acid compounds, e.g. Tinopal DMS pure Xtra and Blankophor (Trade Mark) HRH, and Pyrazoline compounds, e.g. Blankophor SN. Preferred fluorescers are: sodium 2 (4-styryl-3-sulfophenyl)-2H-napthol[1 ,2-d]triazole, disodium 4,4'-bis{[(4-anilino-6-(N methyl-N-2 hydroxyethyl) amino 1 ,3,5-triazin-2-yl)]amino}stilbene-2-2' disulfonate, disodium 4,4'-bis{[(4-anilino-6-morpholino-1 ,3,5-triazin-2-yl)]amino} stilbene-2-2' disulfonate, and disodium 4,4'-bis(2-sulfostyryl)biphenyl.

It is preferred that the aqueous solution used in the method has a fluorescer present. When a fluorescer is present in the aqueous solution used in the method it is preferably in the range from 0.0001 g/l to 0.1 g/l, preferably 0.001 to 0.02 g/l.

### Dye

The composition preferably comprises a dye. Dyes are discussed in K.Hunger (ed). Industrial Dyes: Chemistry, Properties, Applications (Weinheim: Wiley-VCH 2003). Organic dyes are listed in the colour index (Society of Dyers and Colourists and the American Association of Textile Chemists and Colorists)
Preferred dye chromophores are azo, azine, anthraquinone, phthalocyanine and triphenylmethane.

Azo, anthraquinone, phthalocyanine and triphenylmethane dyes preferably carry a net anionic charged or are uncharged. Azine dyes preferably carry a net anionic or cationic charge.

Preferred non-shading dyes are selected are selected from blue dyes, most preferably anthraquinone dyes bearing sulphonate groups and triphenylmethane dye bearing sulphonate groups. Preferred compounds are acid blue 80, acid blue 1, acid blue 3; acid blue 5, acid blue 7, acid blue 9, acid blue 1 1, acid blue 13, acid blue 15, acid blue 17, acid blue 24, acid blue 34, acid blue 38, acid blue 75, acid blue 83, acid blue 91, acid blue 97, acid blue 93, acid blue 93:1, acid blue 97, acid blue 100, acid blue 103, acid blue 104, acid blue 108, acid blue 109, acid blue 1 10, and acid blue 213. On dissolution granules with non-shading dyes provide an attractive colour to the wash liquor.

Blue or violet Shading dyes are most preferred. Shading dyes deposit to fabric during the wash or rinse step of the washing process providing a visible hue to the fabric. In this regard the dye gives a blue or violet colour to a white cloth with a hue angle of 240 to 345, more preferably 260 to 320, most preferably 270 to 300. The white cloth used in this test is bleached non-mercerised woven cotton sheeting.

Shading dyes are discussed in WO 2005/003274, WO 2006/032327(Unilever), WO 2006/032397(Unilever), WO 2006/045275(Unilever), WO 2006/027086(Unilever), WO 2008/017570(Unilever), WO 2008/141880(Unilever), WO 2009/132870(Unilever), WO 2009/141 173 (Unilever), WO 2010/099997(Unilever), WO 2010/102861 (Unilever), WO 2010/148624(Unilever), WO 2008/087497 (P&G), WO 2011/011799 (P&G), WO 2012/054820 (P&G), WO 2013/142495 (P&G) and WO 2013/151970 (P&G).

A mixture of shading dyes may be used.

The shading dye chromophore is most preferably selected from mono-azo, bis-azo, anthraquinone, and azine.

Mono-azo dyes preferably contain a heterocyclic ring and are most preferably thiophene dyes. The mono-azo dyes are preferably alkoxylated and are preferably uncharged or anionically charged at pH=7. Alkoxylated thiophene dyes are discussed in WO 2013/142495 and WO 2008/087497.

Most preferred shading dyes are selected from Direct Violet 9, Direct Violet 99, Direct Violet 35, Solvent Violet 13, Disperse Violet 28, dyes of the structure

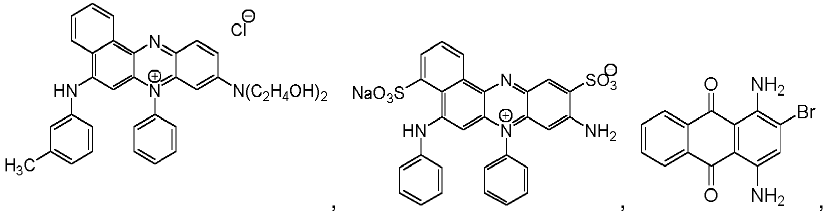

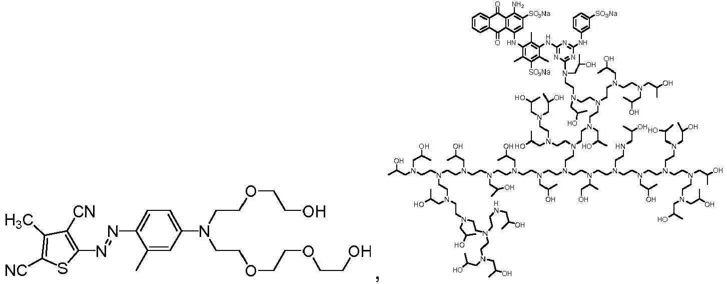

### Perfume

Preferably the composition comprises a perfume. The perfume is preferably in the range from 0.001 to 3 wt.%, most preferably 0.1 to 1 wt.%. Many suitable examples of perfumes are provided in the CTFA (Cosmetic, Toiletry and Fragrance Association) 1992 International Buyers Guide, published by CFTA Publications, and OPD 1993 Chemicals Buyers Directory 80th Annual Edition, published by Schnell Publishing Co.

It is commonplace for a plurality of perfume components to be present in a formulation. In the compositions of the present invention it is envisaged that there will be four or more, preferably five or more, more preferably six or more or even seven or more different perfume components.

In perfume mixtures preferably 15 to 25 wt.% are top notes. Top notes are defined by Poucher (Journal of the Society of Cosmetic Chemists 6(2):80 [1955]). Preferred top-notes are selected from citrus oils, linalool, linalyl acetate, lavender, dihydromyrcenol, rose oxide and cis-3-hexanol.

It is preferred that the laundry treatment composition does not contain a peroxygen bleach, e.g., sodium percarbonate, sodium perborate, and peracid.

### Polymers

The composition may comprise one or more further polymers. Examples are carboxymethylcellulose, poly(ethylene glycol), polyvinyl alcohol), polycarboxylates such as polyacrylates, maleic/acrylic acid copolymers and lauryl methacrylate/acrylic acid copolymers. Polymers present to prevent dye deposition, for example poly(vinylpyrrolidone), poly(vinylpyridine-N-oxide), and poly(vinylimidazole), may be present in the formulation.

Thickening polymers such as anionic acrylic polymers may be included, examples include Acusol 820.

### Enzymes

These materials may be particularly useful in liquid laundry detergent compositions for hand wash.

One or more enzymes are preferred to be present in a cleaning composition of the invention and when practicing a method of the invention.

Preferably the level of each enzyme in the laundry composition of the invention is from 0.0001 wt.% to 0.1 wt.% protein.

Especially contemplated enzymes include proteases, alpha-amylases, cellulases, lipases, peroxidases/oxidases, pectate lyases, and mannanases, or mixtures thereof.

Suitable lipases include those of bacterial or fungal origin. Chemically modified or protein engineered mutants are included. Examples of useful lipases include lipases from Humicola (synonym Thermomyces), e.g. from H. lanuginosa (T. lanuginosus) as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g. from B. subtilis (Dartois et al. (1993), Biochemica et Biophysica Acta, 1 131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202, WO 00/60063.

Preferred commercially available lipase enzymes include Lipolase^{™} and Lipolase Ultra^{™}, Lipex^{™} and lipoclean^{™} (Novozymes A/S).

The method of the invention may be carried out in the presence of phospholipase classified as EC 3.1.1 .4 and/or EC 3.1.1 .32. As used herein, the term phospholipase is an enzyme which has activity towards phospholipids.

Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may be esterified to an amino-alcohol. Phospholipases are enzymes which participate in the hydrolysis of phospholipids. Several types of phospholipase activity can be distinguished, including phospholipases Ai and A2 which hydrolyze one fatty acyl group (in the sn-1 and sn-2 position, respectively) to form lysophospholipid; and lysophospholipase (or phospholipase B) which can hydrolyze the remaining fatty acyl group in lysophospholipid.

Phospholipase C and phospholipase D (phosphodiesterases) release diacyl glycerol or phosphatidic acid respectively.

The enzyme and the photobleach may show some interaction and should be chosen such that this interaction is not negative. Some negative interactions may be avoided by encapsulation of one or other of enzyme or photobleach and/or other segregation within the product.

Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Preferred commercially available protease enzymes include Alcalase^{™}, Savinase^{™}, Primase^{™}, Duralase^{™}, Dyrazym^{™}, Esperase^{™}, Everlase^{™}, Polarzyme^{™}, and Kannase^{™}, (Novozymes A/S), Maxatase^{™}, Maxacal^{™}, Maxapem^{™}, Properase^{™}, Purafect^{™}, Purafect OxP^{™}, FN2^{™}, and FN3^{™} (Genencor International Inc.).

The method of the invention may be carried out in the presence of cutinase classified in EC 3.1.1 .74. The cutinase used according to the invention may be of any origin.

Preferably cutinases are of microbial origin, in particular, of bacterial, of fungal or of yeast origin.

Suitable amylases (alpha and/or beta) include those of bacterial or fungal origin.

Chemically modified or protein engineered mutants are included. Amylases include, for example, alpha-amylases obtained from Bacillus, e.g. a special strain of B. lichen iformis, described in more detail in GB 1,296,839, or the Bacillus sp. strains disclosed in WO 95/026397 or WO 00/060060. Commercially available amylases are Duramyl^{™}, Termamyl^{™}, Termamyl Ultra^{™}, Natalase^{™}, Stainzyme^{™}, Fungamyl^{™} and BAN^{™} (Novozymes A/S), Rapidase^{™} and Purastar^{™} (from Genencor International Inc.).

Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, Thielavia terrestris, Myceliophthora thermophila, and Fusarium oxysporum disclosed in US 4,435,307, US 5,648,263, US 5,691 ,178, US 5,776,757, WO 89/09259, WO 96/029397, and WO 98/012307.

Commercially available cellulases include Celluzyme^{™}, Carezyme^{™}, Celluclean^{™},
Endolase^{™}, Renozyme^{™} (Novozymes A/S), Clazinase^{™} and Puradax HA^{™} (Genencor International Inc.), and KAC-500(B)^{™} (Kao Corporation).

Suitable peroxidases/oxidases include those of plant, bacterial or fungal origin.

Chemically modified or protein engineered mutants are included. Examples of useful peroxidases include peroxidases from Coprinus, e.g. from C. cinereus, and variants thereof as those described in WO 93/24618, WO 95/10602, and WO 98/15257.

Commercially available peroxidases include Guardzyme^{™} and Novozym^{™} 51004 (Novozymes A/S).

Further enzymes suitable for use are discussed in WO 2009/087524, WO 2009/090576, WO 2009/107091, WO 2009/111258 and WO 2009/148983.

### Enzyme Stabilizers

Any enzyme present in the composition may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

Where alkyl groups are sufficiently long to form branched or cyclic chains, the alkyl groups encompass branched, cyclic and linear alkyl chains. The alkyl groups are preferably linear or branched, most preferably linear.

The indefinite article "a" or "an" and its corresponding definite article "the" as used herein means at least one, or one or more, unless specified otherwise.

The invention will be further described with the following non-limiting examples.

### Examples

An Expert Panel Assessment was carried out with the objective of defining and measuring how products differ in specific sensory characteristics compared to control formulations. This was carried out through blind testing of the products. The study carried out ensured all panelists saw and assessed all the Prototypes and the Control Products in randomized order. The panelists are trained panelists: these are panelists who have been screened for their sensory acuity and trained profiling technique of which there were between 12 and 15 panelists.

The panel works with a 0-10 scale, with two anchor points, 1 being not very mild and 9 being very mild.

After the washing process, the panelist waited for 10 minutes while their hands drip-dried. They then assessed their hands for mild feel.

For the mildness attributes the following sensorial attributes was measured:
**Mild on hands feel after drying hands** is the perceived creamy/layer impression on the hands after drying the hand; assessment by slipping between the hand surface with the fingers and feeling with the thumb over the other fingers and is again measured on a ranking scale between not at all mild and very mild.

### Formulations used - controls

| **Ingredient** | **Control 1 (wt.%)** | **Control 2 (wt.%)** | **Control 3 (wt.%)** |
|---|---|---|---|
| LAS | 11.000 | 7.000 | 5.200 |
| SLES 1EO | 5.000 | 7.000 | 9.300 |
| Polyox WSR 301 (Polyethylene glycol) | | 0.005 | 0.005 |
| MGSO₄.7H₂O | 2.500 | 3.700 | 3.200 |
| Microcare IT (preservative) | 0.0009 | 0.0009 | 0.0009 |
| Glydant (preservative) | 0.220 | 0.220 | 0.220 |
| EDTA | 0.100 | 0.100 | 0.100 |
| Water | to 100 | to 100 | to 100 |
| pH | 5.55 | 5.40 | 5.40 |

| | | | |
|---|---|---|---|
| The control formulations all had acceptable viscosity of between 1180 and 1610 centipoise (cP) (in metric units between 1.18 to 1.610 Pa*s) Viscosity was measured at 25°C using the Anton Paar ASC rheometer - using a Bob set-up and reporting the viscosity measured at a shear rate of 23s⁻¹ | | | |

### Formulations used - according to the invention

| **Ingredient** | **1 (wt.%)** | **2 (wt.%)** | **3 (wt.%)** | **4 (wt.%)** | **5 (wt.%)** |
|---|---|---|---|---|---|
| LAS | 5.800 | 5.000 | 4.800 | 4.160 | 5.400 |
| SLES 1EO | 5.800 | 5.000 | 4.800 | 7.440 | 5.400 |
| Rhamnolipid R2 | 2.900 | 2.500 | 2.900 | 2.900 | 2.700 |
| CAPB | | | 2.000 | | |
| MGSO₄.7H₂O | 1.500 | 2.500 | 1.300 | 1.800 | 1.7000 |
| Acusol 820 (thickener) | 0.500 | 0.600 | | | 0.600 |
| Microcare IT (preservative) | 0.0009 | 0.0009 | 0.0009 | 0.0009 | 0.0009 |
| Glydant (preservative) | 0.220 | 0.220 | 0.220 | 0.220 | 0.220 |
| EDTA | 0.100 | 0.100 | 0.100 | 0.100 | 0.100 |
| Water | to 100 | to 100 | to 100 | to 100 | to 100 |
| pH | 5.42 | 5.57 | 6.02 | 5.83 | 5.53 |

| | | | | | |
|---|---|---|---|---|---|
| Rhamnolipid R2 is a Di Rhamnolipid supplied from Evonik. The formulations according to the invention all had acceptable viscosity of between 1350 and 1520 centipoise (cP) (in metric units between 1.35 to 1.52 Pa*s). Viscosity was measured at 25°C using the Anton Paar ASC rheometer - using a Bob set-up and reporting the viscosity measured at a shear rate of 23s⁻¹ | | | | | |

### Results tables for long lasting mildness attribute

### 1 versus controls 1 to 3 for long lasting mildness

| **Attribute** | **1** | **Control 1** | **Control 2** | **Control 3** |
|---|---|---|---|---|
| Mild on hands after drying | 6.00 | 3.96 | 4.14 | 3.57 |

| | | | | |
|---|---|---|---|---|
| Formulation 1 gave statistically significant (Tukey's HSD Test with 95% confidence interval) improvement of the attribute versus controls 1 to 3 | | | | |

### 2 versus controls 1 to 3 for long lasting mildness

| **Attribute** | **2** | **Control 1** | **Control 2** | **Control 3** |
|---|---|---|---|---|
| Mild on hands after drying | 5.11 | 3.96 | 4.14 | 3.57 |

| | | | | |
|---|---|---|---|---|
| Formulation 2 gave statistically significant (Tukey's HSD Test with 95% confidence interval) improvement of the attribute versus controls 1 to 3 | | | | |

### 3 versus controls 1 to 3 for long lasting mildness

| **Attribute** | **3** | **Control 1** | **Control 2** | **Control 3** |
|---|---|---|---|---|
| Mild on hands after drying | 5.12 | 3.96 | 4.14 | 3.57 |

| | | | | |
|---|---|---|---|---|
| Formulation 3 gave statistically significant (Tukey's HSD Test with 95% confidence interval) improvement of the attribute versus controls 1 to 3 | | | | |

### 4 versus controls 1 to 3 for long lasting mildness

| **Attribute** | **4** | **Control 1** | **Control 2** | **Control 3** |
|---|---|---|---|---|
| Mild on hands after drying | 5.96 | 3.96 | 4.14 | 3.57 |

| | | | | |
|---|---|---|---|---|
| Formulation 4 gave statistically significant (Tukey's HSD Test with 95% confidence interval) improvement of the attribute versus controls 1 to 3 | | | | |

### 5 versus controls 1 to 3 for long lasting mildness

| **Attribute** | **5** | **Control 1** | **Control 2** | **Control 3** |
|---|---|---|---|---|
| Mild on hands after drying | 5.06 | 3.96 | 4.14 | 3.57 |

| | | | | |
|---|---|---|---|---|
| Formulation 5 gave statistically significant (Tukey's HSD Test with 95% confidence interval) improvement of the attribute versus controls 1 to 3 | | | | |

As can be seen from the data, the use of a rhamnolipid in a surfactant system for handwash detergents confers to the consumer a sensorial impression of mildness on the hands. This sensorial impression is particularly seen where the mildness on the hands lasts after the hands are subsequently dried after the handwash process is ended.

## Claims

1. Use of a rhamnolipid in a surfactant system for handwash detergents to confer to the consumer a sensorial impression of mildness on the hands-, wherein the rhamnolipid is present in the surfactant system at a level of from 15 to 50 wt.% of the surfactant system, wherein the use involves intimate contact of the detergent liquor with the hands during the washing process, whether in laundry processes or hand dish wash processes.

2. Use according to claim 1, wherein the sensorial impression of mildness on the hands lasts after the hands are subsequently dried after the handwash process is ended.

3. Use according to any preceding claim, wherein the handwash detergent is a fluid detergent composition, preferably an aqueous detergent composition.

4. Use according to any preceding claim, wherein the handwash detergent is a hand dish wash composition, or liquid laundry detergent composition for hand wash.

5. Use according to claim 4, wherein the rhamnolipid is present in the composition at a level of from 1 to 20 wt.%, preferably from 1.25 to 15 wt.%, more preferably from 1.5 to 12.5 wt.%, most preferably from 2 to 10 wt.%.

6. Use according to any preceding claim, wherein the rhamnolipid comprises at least 50 wt.% di-rhamnolipid, more preferably at least 60 wt.% di-rhamnolipid, even more preferably 70 wt.% di-rhamnolipid, most preferably at least 80 wt.% di-rhamnolipid.

7. Use according to any preceding claim, wherein the rhamnolipid is a di-rhamnolipid of formula: Rha2C₈₋₁₂C₈₋₁₂.

## Patentansprüche

1. Verwendung eines Rhamnolipids in einem Tensidsystem für Handwaschmittel, um dem Verbraucher einen sensorischen Eindruck von Milde auf den Händen zu vermitteln, wobei das Rhamnolipid in dem Tensidsystem in einer Menge von 15 bis 50 Gew.-% des Tensidsystems vorhanden ist, wobei die Verwendung den innigen Kontakt der Waschlauge mit den Händen während des Waschverfahrens, ob im Wäschewasch- oder Handgeschirrspülverfahren, einschließt.

2. Verwendung nach Anspruch 1, wobei der sensorische Eindruck der Milde auf den Händen, nachdem die Hände nach dem Handwaschverfahren getrocknet sind, anhält.

3. Verwendung nach einem vorhergehenden Anspruch, wobei das Handwaschmittel eine flüssige Waschmittelzusammensetzung ist, vorzugsweise eine wässrige Waschmittelzusammensetzung.

4. Verwendung nach einem vorhergehenden Anspruch, wobei das Handwaschmittel eine Handgeschirrspülzusammensetzung oder eine flüssige Wäschewaschmittelzusammensetzung zum Handwaschen ist.

5. Verwendung nach Anspruch 4, wobei das Rhamnolipid in der Zusammensetzung in einer Menge von 1 bis 20 Gew.-%, vorzugsweise von 1,25 bis 15 Gew.-%, besonders bevorzugt von 1,5 bis 12,5 Gew.-%, höchst bevorzugt von 2 bis 10 Gew.-%.

6. Verwendung nach einem vorhergehenden Anspruch, wobei das Rhamnolipid mindestens 50 Gew.-% Di-rhamnolipid, besonders bevorzugt mindestens 60 Gew.-% Di-rhamnolipid, noch bevorzugter mindestens 70 Gew.-% Di-rhamnolipid, höchst bevorzugt mindestens 80 Gew.-% Di-rhamnolipid, umfasst.

7. Verwendung nach einem vorhergehenden Anspruch, wobei das Rhamnolipid ein Di-rhamnolipid der Formel Rha2C₈₋₁₂C₈₋₁₂ ist.

## Revendications

1. Utilisation d'un rhamnolipide dans un système tensioactif pour détergents de lavage à la main pour conférer au consommateur une impression sensorielle de douceur sur les mains, dans laquelle le rhamnolipide est présent dans le système tensioactif à un niveau de 15 à 50 % en poids du système tensioactif, dans laquelle l'utilisation implique le contact intime de la lessive détergente avec les mains pendant le procédé de lavage, que ce soit dans des procédés de lavage du linge ou des procédés de lavage de la vaisselle à la main.

2. Utilisation selon la revendication 1, dans laquelle l'impression sensorielle de douceur sur les mains dure après que les mains sont ensuite séchées après que le procédé de lavage à la main est terminé.

3. Utilisation selon une quelconque revendication précédente, dans laquelle le détergent de lavage à la main est une composition détergente fluide, de préférence une composition détergente aqueuse.

4. Utilisation selon une quelconque revendication précédente, dans laquelle le détergent de lavage à la main est une composition de lavage de la vaisselle à la main, ou une composition détergente liquide pour le lavage du linge à la main.

5. Utilisation selon la revendication 4, dans laquelle le rhamnolipide est présent dans la composition à un niveau de 1 à 20 % en poids, de préférence de 1,25 à 15 % en poids, de manière davantage préférée de 1,5 à 12,5 % en poids, de manière préférée entre toutes de 2 à 10 % en poids.

6. Utilisation selon une quelconque revendication précédente, dans laquelle le rhamnolipide comprend au moins 50 % en poids de di-rhamnolipide, de manière davantage préférée au moins 60 % en poids de di-rhamnolipide, de manière encore davantage préférée 70 % en poids de di-rhamnolipide, de manière préférée entre toutes au moins 80 % en poids de di-rhamnolipide.

7. Utilisation selon une quelconque revendication précédente, dans laquelle le rhamnolipide est un di-rhamnolipide de formule : Rha2C₈₋₁₂C₈₋₁₂.
